# EUROPÄISCHE PATENTSCHRIFT

(11) **EP 4 153 986 B1**
(45) Veröffentlichungstag und Bekanntmachung des Hinweises auf die Patenterteilung: **26.06.2024**
(21) Anmeldenummer: 21732182.7
(22) Anmeldetag: 17.05.2021
(51) Int. Cl.: A61L 9/22, G01N 27/64, H01J 1/35, H01J 3/02

(54) **GASSENSOR MIT EINER ELEKTRONENEMITTERSTRUKTUR**
GAS SENSOR COMPRISING AN ELECTRON EMITTER STRUCTURE
CAPTEUR DE GAZ COMPRENANT UN ÉMETTEUR D'ÉLECTRONS

(30) Priorität: 18.05.2020 DE 102020113351
(43) Veröffentlichungstag der Anmeldung: 29.03.2023
(73) Patentinhaber: DBT GmbH, 79070 Würzburg (DE)
(72) Erfinder: DOLL, Theodor, 30916 Isernhagen (DE)
(74) Vertreter: Weidner Stern Jeschke
(86) Internationale Anmeldenummer: PCT/DE2021/100436
(87) Internationale Veröffentlichungsnummer: WO 2021/233501

(56) Entgegenhaltungen:
- EP-A1- 1 026 721
- EP-A1- 1 320 116
- EP-A2- 0 416 626
- WO-A1-01/26134
- WO-A1-02/05305
- WO-A1-02/15223
- WO-A1-2019/208328
- DE-A1-102011 013 262
- JP-A- H04 152 296
- JP-A- 2018 041 736
- US-A- 4 370 797
- US-A1- 2006 090 996
- BEGLEY P ET AL: "Photoemissive ionisation source for ion mobility detectors", JOURNAL OF CHROMATOGRAPHY A, ELSEVIER, AMSTERDAM, NL, Bd. 588, Nr. 1-2, 27. Dezember 1991 (1991-12-27), Seiten 239-249, XP026509018, ISSN: 0021-9673, DOI: 10.1016/0021-9673(91)85029-F [gefunden am 1991-12-27]
- JUNG HAN-IL ET AL: "Low-voltage and low-power field-ionization gas sensor based on micro-gap between suspended silver nanowires electrodes for toluene detection", 2017 IEEE 30TH INTERNATIONAL CONFERENCE ON MICRO ELECTRO MECHANICAL SYSTEMS (MEMS), IEEE, 22. Januar 2017 (2017-01-22), Seiten 195-198, XP033069538, DOI: 10.1109/MEMSYS.2017.7863374 [gefunden am 2017-02-23]

## Beschreibung

Die vorliegende Erfindung betrifft einen Gassensor mit einem Emitter.

Elektronen niedriger Energie werden beispielsweise zur Luftreinigung oder zur Spurengasanalyse verwendet. Zur Bildung von Ionen an der Luft benötigt man Ladungen. Diese entstehen durch Bestrahlung der Luftmoleküle mit energiereicher elektromagnetischer Strahlung, durch radioaktive Quellen oder mittels Elektronenquellen. Die Ladungen gehen Bindungen mit den Luftmolekülen ein. Bei Ladungserzeugung mittels energiereicher elektromagnetischer Strahlung und durch radioaktive Quellen bilden sich vielfältige Ionenarten aufgrund von hohen und unscharfen Energien bei der Ladungserzeugung oder auch durch Ladungsweitergabe. Für die Bildung von Ionen in Luft mittels Elektronenquellen werden Elektronen in die Luftmoleküle abgegeben. Elektronen niedriger Energie können diese Moleküle ionisieren. Die derart gebildeten Ionen lassen sich in einem elektrischen Feld einfangen und so der Umgebungsluft entziehen. Hierdurch lassen sich beispielsweise Staubteilchen, schädliche Stoffe, Verschmutzungen, sowie Milben, Pollen, Gerüche, Krankheitskeime und Bakterien binden. Derartige Elektronenquellen lassen sich daher in Luftreinigern einsetzen. Ob die Elektronen von den Molekülen eingefangen werden können hängt im Wesentlichen von der Energie der Elektronen ab. Unterschiedliche Moleküle können Elektronen verschiedener Energie einfangen. Die Aufladung von nur bestimmten Molekülen ist durch Elektronen mit einer sehr scharfen Energieverteilung in einem niedrigen Energiebereich möglich. Derartige Elektronen eignen sich somit auch zur Ionisation von Molekülen zur Spurengasanalyse, beispielsweise durch Massenspektrometrie.

### Stand der Technik

Bekannt ist die Ionenbildung mit UV-Quellen in Photoionisationsdetektoren (PID-Sensoren), die Ionisation mit ⁶³Ni-Quellen in Analysengeräten oder auch die Elektronenstoßionisation im Vakuum bei Massenspektrometern. UV-Quellen werden auch in Luftreinigern zur Ionisation eingesetzt. Hierbei entsteht außerdem Ozon, das auch einen Teil zur Luftreinigung beiträgt.

Allerdings bergen die Abbauprodukte von Nikotin und Zigarettenrauch mit dem Ozon, neben dem Ozon selbst, hohe gesundheitliche Risiken. Ozon generierende Luftreiniger sind daher nachteilig.

UV-Quellen in PID-Sensoren ionisieren über den Compton-Effekt alle Moleküle, deren Ionisierungsenergie E unter der Photonenenergie hv liegt. Hierdurch wird ein breites Spektrum verschieden stark ionisierter Moleküle erzeugt, dass für Präzisionsanwendungen, wie beispielsweise die oben genannte Spurengasanalyse weniger gut geeignet ist.

Freie Elektronen an Luft lassen sich durch Feldemission oder Thermoemission erzeugen. Bei der Feldemission werden Elektronen durch ein starkes elektrisches Feld unter Ausnutzung des Tunneleffekts aus einer negativ geladenen Elektrode gelöst. Thermoemission bezeichnet den thermisch induzierten Fluss von Elektronen von einer Oberfläche oder über eine Potentialbarriere. Hierbei überwinden die Elektronen im Träger die Austrittsarbeit des Materials durch ihnen zugeführte Wärmeenergie. Thermoemission ist unter atmosphärischen Bedingungen schwieriger zu erzeugen als Feldemission, ließe sich aber mit hochtemperaturfesten, oxidationsstabilen Materialien wie beispielsweise SnO₂ oder Antimondotiertem SnO₂ erzeugen.

Außerdem lassen sich Elektronen durch den äußeren Photoeffekt erzeugen. Hierbei wird ein Elektron durch Absorption eines Photons aus einer Bindung aus einem Festkörper gelöst. Aus dem Stand der Technik ist ein sogenannter Elektronen-Ionisationsdetektor ("ePID") bekannt, der auf der Basis des äußeren Photoeffektes Elektronen sehr niedriger Energie aus einem Emittermaterial generiert und diese auf Weglängen der Größenordnung einiger mittlerer freier Weglängen der Moleküle bei Normalbedingungen in einem elektrischen Feld beschleunigt. Damit wird theoretisch die Elektronenenergie frei einstellbar, wodurch unterschiedliche Spurengase in Luft nicht nur summenmengenmäßig sondern auch anhand ihrer Ionisierungsenergien in gewissem Masse identifiziert werden können. Zur Elektronenfreisetzung werden UV-Licht emittierende Quellen wie z.B. Dioden (UV-LEDs) verwendet.

Grundsätzlich sind emissive Materialien mit geringen Austrittsarbeiten am besten geeignet, freie Elektronen an Luft zu erzeugen. Diese Materialien weisen eine hohe Dichte emittierbarer Elektronen auf, d.h. sie weisen eine hohe Elektronendichte am Ferminiveau bzw. an der Valenzbandkante auf. Die bisher für diesen Zweck eingesetzten Materialien niedriger Austrittsarbeit von etwa 1-2 eV wie Ba, BaO, Cs, also Alkali- und Erdalkalimetalle und ihre halbleitenden Oxide sind an Luft aber hochreaktiv, d.h. sie oxidieren exotherm oder bilden ein Hydroxid. Sie sind daher nur unter Hochvakuumbedingungen einsetzbar. Auch Lanthanborid, das beispielsweise in Feldemitterspitzen von Elektronenmikroskopen zur Elektronenemission eingesetzt wird, eignet sich nicht für Anwendungen unter atmosphärischen Bedingungen, da es vor einer Verwendung als Elektronenemitter mit hohen Temperaturen im Vakuum von seinem Oberflächenoxid befreit werden muss. Eine Anwendung an Luft führt zur Re-oxidation und wäre somit nicht, oder nur für sehr kurze Zeit (etwa für wenige µs) möglich.

Edelmetalle wie z.B. Gold, Platin und Silber hingegen sind zwar gegenüber einer Oxidation stabil bzw. bilden nur dünnste Oxid- oder Sulfidschichten, welche leicht durchtunnelt werden können, haben aber sehr hohe Austrittsarbeiten im Bereich von 4,8 bis 5,6 eV. Dieser Energiebereich ist für UV-LEDs bis heute nicht zugänglich. Ähnlich verhält es sich mit den Metallen, die "gediegen" vorkommen, also als reine Metalle mit nur leichten oxidischen Überzügen. Zu dieser Gruppe gehören Arsen, Antimon, Tellur, Blei, Wismut, Indium und Zinn. Sie haben meist Austrittsarbeiten von mehr als 4 eV und ihre Oxide weisen vergleichbare Werte auf. Die Nebengruppenmetalle, insbesondere Ventilmetalle, deren Austrittsarbeiten etwa 1 eV niedriger als die der gediegenen Metalle liegen, bilden meist isolierende, elektronenarme Oxide mit Dicken, die über der üblichen Tunneldistanz von 5 nm liegen. Die Austrittsarbeiten ihrer Oxide liegen meist nicht nennenswert unter denen der Reinmetalle, wenngleich einige als Halbleiter (NiO, CuOₓ, CoOₓ, Cr₂O₃, SnO₂) zumindest Leitungselektronen aufweisen.

Lanthan und ähnliche Seltenerdmetalle der gleichen Gruppe weisen, wegen ihrer Nähe zu den Erdalkalimetallen, einen niedrigeren und damit geeigneteren Austrittsarbeitsbereich von 2,5 bis 3,6 eV auf und ihre Oxide ebenso. Diese Materialien sind aber nicht stabil, wenn sie Luftfeuchtigkeit ausgesetzt sind (sie bilden Hydroxide und oxidieren vollständig über ihr gesamtes Volumen).

Eine Ausnahme mit einzigartigen Eigenschaften bildet Thorium mit einer Austrittsarbeit von 2,2-2,4 eV, einer Austrittsarbeit des Oxids von 2,8 eV und Stabilität gegenüber den Luftbestandteilen. Aufgrund seiner Radioaktivität, besonders aber wegen der hohen Energie der emittierten Alphateilchen von 54 MeV, welche unspezifisch ionisieren, kommt dieses Material für Anwendungen, die eine niedrige, scharfe Elektronenenergie erfordern nicht infrage. Cer, Samarium und Ytterbium (und die normalen Nebengruppenelemente Yttrium und Strontium), werden in anderen Anwendungen als Thoriumersatz verwendet. Diese unterliegen als reine Metalle aber insofern den Limitationen der Lanthanide, dass sie schnell oxidieren und mit Wasser reagieren und kommen daher ebenfalls nicht infrage.

Die aus dem Stand der Technik bekannten ePID-Entwürfe können daher nicht langzeitstabil unter atmosphärischen Bedingungen betrieben werden. Hierdurch ist ein Einsatz in elektrostatisch betriebenen Luftreinigern, die ozonfrei arbeiten nicht möglich. Auch Analysemethoden wie Massenspektroskopie ist mit den aus dem Stand der Technik bekannten ePID-Entwürfen unter atmosphärischen Bedingungen ebenfalls nicht möglich. Wir benötigen dazu freie Elektronen in der Luft mit möglichst genauen (scharfen) Energien im Bereich von z. B. < 25 eV. Ebenso fehlt ein atmosphärenstabiles, d.h. gegenüber Sauerstoff und Feuchte langzeitstabiles, hochemissives Material, welches eine möglichst niedrige Austrittsarbeit aufweist. Ein solches Material würde die Verwendung kostengünstiger Lichtquellen (z.B. UV-LED) für den äußeren Photoeffekt erlauben.

DE 10 2011 013 262 A1 offenbart Elektronen-lonisationsquelle, wobei Photoelektronen aus einer atmosphärenstabilen Schicht durch eine UV-Quelle erzeugt und mit einer Beschleunigungsstrecke in der Größenordnung der mittleren freien Weglänge der Gase oder kleiner auf die lonisationsenergien der Gase gebracht werden.

Ein Elektronenemissionsmaterial ist in der JP 04152296 A offenbart.

In der US 2006/0090996 A1 offenbart einen photokatalytischen Reiniger für Luft oder Wasser mit einer Anode, die einen Leuchtstoff enthält, der als Reaktion auf ein elektrisches Feld Elektronen aussendet.

Die Kombination aus einer photoemittierenden Ionisationsquelle und eines Ionenmobilitätsdetektor unter Verwendung einer gepulsten Lichtquelle, wodurch die Notwendigkeit einer Ioneninjektionsschleuse entfällt, wurde in dem Beitrag "Photoemissive ionisation source for ion mobility detectors" von P. Begley, R. Corbin, B. E. Foulger* and P. G. Simmonds im Journal of Chromatography, 588 (1991) 239-249 veröffentlicht.

JP 2018041736A offenbart eine Ladevorrichtung von in Luft oder Gas dispergierten feinen Teilchen und eine Vorrichtung zur Analyse der geladenen feinen Teilchen, z.B. ein Massenspektrometer.

Eine Feldelektronenemissionsquelle, welche auf einem Siliziumsubstrat vom n-Typ eine Driftschicht mit starkem Feld und eine aus einem dünnen Goldfilm bestehende Oberflächenelektrode aufweist, ist in der EP 1320116A1 offenbart.In der WO 02/05305 A1 ist eine Anordnung zur Emission von Elektronen für die Elektronenstrahlphotolithographie und Bildschirme offenbart, wobei eine Emittervorrichtung einen erste Leiter, der sich mit parallelen, beabstandeten zweiten Leitern kreuzt, aufweist.

In dem Konferenzbeitrag "Low-voltage and low-power field-ionization gas sensor based on micro-gap between suspended silver nanowires electrodes for toluene detection" bei der 30th IEEE International Conference on Micro Electro Mechanical Systems, MEMS 2017 - Las Vegas, United States (Proceedings DOI: 10.1109/MEMSYS.2017.7863374 S. 195.108) beschreiben Han Il Jung, Soonjae Pyo, Jongbaeg Kim einen Feldionisations-Gassensor mit schwebenden Silber-Nanodrähten als Elektroden.

EP 102673121 A1 offenbart eine Feldemissions-Elektronenquellen und ein Verfahren zur Herstellung des Arrays, das Elektronen von gewünschten Bereichen einer Oberflächenelektrode von Feldemissions-Elektronenquellen entlädt.

Die EP 0416626 A2 offenbart eine Elektronen emittierende Halbleitervorrichtung mit einer P-Halbleiterschicht, die auf einem halbleitenden Substrat ausgebildet ist, einer Shottky-Barrieren-Elektrode, die auf der P-Halbleiterschicht ausgebildet ist, mehreren P-Flächeneinheiten, die unter der Shottky Barriere-Elektrode angeordnet sind und einem N-Bereich in der Nähe der P-Bereichseinheiten.

Ein Halbleiterbauelement zum Erzeugen eines Elektronenstrahls durch den Avalanche-Effekt in einem p-n-Übergang, der sich parallel zur Oberfläche des Halbleitervolumens in Sperrichtung eines vorgespannten p-n-Übergang erstreckt ist in der US 4270797 A offenbart. Die WO 02/15223 A1 offenbart ein Elektronenstrahllithographiesystem mit hohem Durchsatz bei kürzerer Elektronenstrahlsäulenlänge, reduzierten Elektron-Elektronen-Wechselwirkungen und höherem Strahlstrom. Das System umfasst eine Photokathode mit einem Muster, das sich aus einer periodischen Anordnung von Öffnungen mit einer bestimmten Geometrie zusammensetzt.

Ein lithographischer Apparat, der eine Anordnung von Säulen mit geladenen Teilchenstrahlen (Elektronenstrahlen) verwendet, wobei die Anordnung eine Vielzahl von Ladungsteilchenstrahlsäulen enthält, die jeweils selektiv ein Ziel mit einer Vielzahl von Ladungsteilchenstrahlen belichten, ist in der WO 01/26134 A1 offenbart.

Die WO2019/208328 A1 offenbart einen Gassensor mit einem Flächenemitter.

Der vorliegenden Erfindung liegt somit die Aufgabe zugrunde, einen Gassensor mit einem effizienten Emitter für Elektronen geringer Energie im Bereich von unter 25 eV mit geringer Energiebreite bereitzustellen, der langzeitstabil betrieben werden kann.

### Beschreibung der Erfindung

Erfindungsgemäß wird diese Aufgabe gelöst durch einen Gassensor gemäß Anspruch 1, insbesondere umfassend einen Tunnel-Flächenemitter, mit einer Elektronenemitterstruktur, eine auf der zweiten Seite der Elektronenemissionsschicht angeordnete Isolatorschicht, die eine Schichtdicke zwischen 0,5 nm und 6 nm aufweist, und eine auf einer der Elektronenemissionsschicht abgewandten Seite auf der Isolatorschicht angeordnete Metallschicht, die eine Schichtdicke zwischen 0,5 nm und 6 nm aufweist, wobei sowohl die Elektronenemissionsschicht als auch die Isolatorschicht durchtunnelbare Schichtdicken aufweisen, umfasst, wobei die Elektronenemitterstruktur: a) eine atmosphärenstabile Elektronenemissionsschicht aus einem Gemisch von Metallen, wobei die atmosphärenstabile Elektronenemissionsschicht eine erste Seite und eine zweiten Seite aufweist; und b) eine Elektronenbeschleunigungsstruktur auf der ersten Seite der Elektronenemissionsschicht mit wenigstens einer gegenüber der Elektronenbeschleunigungsstruktur elektrisch isolierten Elektrode zur Bildung einer Beschleunigungsstrecke, die konfiguriert ist, um zu ermöglichen, dass aus der Elektronenemissionsschicht herausgelöste Elektronen bei Erzeugen eines einstellbaren elektrischen Feldes gezielt beschleunigt werden, wobei die Beschleunigungsstrecke eine Länge l in einem Bereich von 10 nm bis 1 µm aufweist, umfasst und eine Ionendetektionseinrichtung; und einen Druckreduktionsbehälter, in dem der Tunnel-Flächenemitter angeordnet ist, wobei im Betrieb des Gassensors der Druck im Druckreduktionsbehälter relativ zum Umgebungsdruck reduziert ist.

Als Metalle in dem Gemisch von Metallen sind ausschließlich Gemische elementarer Metalle zu verstehen, also Metalle in der Oxidationsstufe 0. Insbesondere Metalloxide werden somit nicht als erfindungsgemäße Metalle verstanden.

Günstigerweise ist das Gemisch von Metallen eine Legierung, vorzugsweise umfassend eine Verbindung ausgewählt aus InCe, AsCeSm, AgSm, AgCe, AgSmCe und AgAsCeSm und/oder zwei oder mehr Elemente aus As, Ag, Zn, Au, Pt, Ru Rh, Pd, Os, Ir, Ce, Zn, Bi, Te, Sm, Eu, Gd, Yt, Yb, Nd, Pr und La.

Alternativ oder zusätzlich umfasst das Gemisch von Metallen ein Eutektikum, umfassend mindestens ein Edelmetall, mindestens ein Lanthanid und mindestens ein Element ausgewählt aus, As, Te, Bi und Sn.

Hinsichtlich des Gemischs von Metallen kann "umfassen" vorzugsweise "bestehen aus" bedeuten.

Als Eutektika werden Materialkombinationen an ihren eutektischen Punkten oder in der Umgebung der eutektischen Punkte verstanden.

Vorteilhafterweise umfasst die Elektronenbeschleunigungsstruktur ferner wenigstens ein isolierendes Strukturelement, das zusammen mit der wenigstens einen Elektrode wenigstens ein Elektronenbeschleunigungselement bildet.

In einer besonderen Ausführungsform der Erfindung ist vorgesehen, dass das wenigstens eine isolierende Strukturelement zwischen der Elektronenemissionsschicht und der wenigstens einen Elektrode angeordnet und damit verbunden ist.

In einer weiteren besonderen Ausführungsform der Erfindung umfasst die Elektronenemitterstruktur mehrere Elektronenbeschleunigungselemente, die in einem Array, vorzugsweise lateral, angeordnet sind.

Es ist möglich, dass die Elektronenemitterstruktur als ein Äußerer-Photoeffekt-Emitter weitergebildet ist, umfassend eine Elektronenemitterstruktur nach einer der zuvor genannten Ausführungsformen, und eine oder mehrere UV-LEDs zum Herauslösen von Elektronen aus der Elektronenemissionsschicht, wobei die eine oder mehrere UV-LEDs konfiguriert ist bzw. sind, um elektromagnetische Strahlung zumindest teilweise auf die Elektronenemissionsschicht zu emittieren. Der Äußerer-Photoeffekt-Emitter ist nicht Teil der vorliegenden Erfindung.

Der Äußerer-Photoeffekt-Emitter umfasst ferner ein Substrat, das auf der zweiten Seite der Elektronenemissionsschicht angeordnet ist, wobei die eine oder mehrere UV-LEDs konfiguriert ist bzw. sind, um elektromagnetische Strahlung zumindest teilweise auf die zweite Seite der Elektronenemissionsschicht zu emittieren und außerhalb und/oder innerhalb des Substrats angeordnet ist bzw. sind, und das Substrat im Emissionswellenlängenbereich der UV-LEDs zumindest teilweise transparent ist.

Günstigerweise umfasst der Äußerer-Photoeffekt-Emitter ferner eine auf einer von der Elektronenemissionsschicht abgewandten Seite des Substrats angeordnete reflektierende Schicht.

Zweckmäßigerweise weist bei dem Äußerer-Photoeffekt-Emitter, die reflektierende Schicht ein Material, ausgewählt aus Platin, Quecksilber, Nickel, Palladium und Iridium auf.

Bei dem Äußerer-Photoeffekt-Emitter ist bzw. sind die eine oder mehreren UV-LEDs konfiguriert, um elektromagnetische Strahlung zumindest teilweise auf die erste Seite der Elektronenemissionsschicht zu emittieren.

Insbesondere kann dabei vorgesehen sein, dass die Elektronenbeschleunigungsstruktur im Emissionswellenlängenbereich der UV-LEDs zumindest teilweise transparent ist.

Zusätzlich kann die Elektronenemitterstruktur zu einer Partikelsammelvorrichtung weitergebildet sein, welche einen Äußerer-Photoeffekt-Emitter nach einem der zuvor genannten Beispiele und zwei mit unterschiedlicher Polarität elektrisch aufladbare Platten zur Erzeugung eines elektrischen Felds aufweist, die auf der ersten Seite der Elektronenemissionsschicht in einem Abstand zueinander und jeweils senkrecht zur Elektronenemissionsschicht angeordnet sind.

Durchtunnelbare Schichtdicken können beispielsweise im Bereich zwischen 0,5 nm und 5 nm liegen. Außerdem kann die Elektronenemitterstruktur zu einem halbleiterbasierten Direktemitter zur Anwendung in der Oberflächenchemie weitergebildet sein, umfassend eine Elektronenemitterstruktur nach einer der zuvor genannten Ausführungsformen , eine Isolatorschicht, die auf der zweiten Seite der Elektronenemissionsschicht angeordnet ist und eine durchtunnelbare Schichtdicke aufweist, und einen auf einer der Elektronenemissionsschicht abgewandten Seite der Isolatorschicht angeordneten Halbleiter, der alternierend nebeneinanderliegende n-dotierte Bereiche und p-dotierte Bereiche aufweist, wobei die n-dotierten Bereiche und die p-dotierten Bereiche an die Isolatorschicht angrenzende Grenzbereiche aufweisen. Der halbleiterbasierte Direktemitter ist nicht Teil der vorliegenden Erfindung.

Günstigerweise umfasst der halbleiterbasierte Direktemitter ferner einen Isolator, der auf einer der Isolatorschicht abgewandten Seite des Halbleiters angeordnet ist, wobei der Isolator Finger aufweist, die an Grenzbereichen zwischen den n-dotierten Bereichen und den p-dotierten Bereichen angeordnet sind und in den Halbleiter hineinragen.

Zusätzlich oder alternativ kann ein vorgenannter halbleiterbasierter Direktemitter eine auf einer der Elektronenemissionsschicht abgewandten Seite der Isolatorschicht angeordnete Lichtemissionsvorrichtung, ausgewählt aus einem VCSEL, einen seitlich emittierenden Halbleiterlaser und/oder eine Leuchtdiode aufweisen.

Ferner kann ein Flüssigkeitsionisator, der nicht Teil der vorliegenden Erfindung ist, weitergebildet sein, umfassend eine halbleiterbasierten Direktemitter und eine auf einer der Elektronenemissionsschicht abgewandten Seite der Elektronenbeschleunigungsstruktur angeordnete Schutzschicht, wobei die Schutzschicht in einer Elektronenemissionsrichtung verlaufende Löcher aufweist.

Vorteilhafterweise weisen die Löcher einen Durchmesser d zwischen 10 µm und 100 µm auf. Es ist ein Verfahren zum Erzeugen von freien Elektronen definierter niedriger Energie mittels eines vorgenannten Äußerer-Photoeffekt-Emitters realisierbar, umfassend die Schritte: Herauslösen von Elektronen aus der Elektronenemissionsschicht unter Verwendung der ein oder mehreren UV-LEDs, und Beschleunigen der herausgelösten Elektronen mittels eines elektrischen Feldes, das durch Anlegen einer Spannung zwischen der Elektronenemissionsschicht und der wenigstens einen Elektrode erzeugt ist.

Ferner ist ein Verfahren zum Sammeln von Partikeln unter Verwendung einer vorgenannten Partikelsammelvorrichtung realisierbar, umfassend die Schritte: Herauslösen von Elektronen aus der Elektronenemissionsschicht, unter Verwendung der ein oder mehreren UV-LEDs, Beschleunigen der herausgelösten Elektronen mittels eines elektrischen Feldes, das durch Anlegen einer Spannung zwischen der Elektronenemissionsschicht und der wenigstens einen Elektrode erzeugt ist, elektrisches Laden von Partikeln durch die beschleunigten Elektronen, und Beschleunigen der elektrisch geladenen Partikel mittels eines zweiten elektrischen Feldes durch Anlegen einer Spannung an den elektrisch aufladbaren Platten.

Außerdem ist ein Verfahren zum Erzeugen von freien Elektronen definierter niedriger Energie mittels eines Tunnel-Flächenemitters nach den genannten Beispielen realisierbar, umfassend die Schritte: Herauslösen von Elektronen aus der Elektronenemissionsschicht durch Anlegen einer Spannung zwischen der Elektronenemissionsschicht und der Metallschicht, und Beschleunigen der herausgelösten Elektronen mittels eines elektrischen Feldes, das durch Anlegen einer Spannung zwischen der Elektronenemissionsschicht und der wenigstens einen Elektrode erzeugt ist.

Weiterhin ist ein Verfahren zum Erzeugen von freien Elektronen definierter niedriger Energie mittels eines der vorgenannten halbleiterbasierten Direktemitters realisierbar, umfassend die Schritte: Erzeugen von Elektronen in einem Grenzbereich zwischen einem n-dotierten Bereich und einem p-dotierten Bereich, Beschleunigen der Elektronen in dem Grenzbereich durch ein von außen angelegtes elektrisches Feld, Erzeugen von herausgelösten Elektronen durch Tunneln der Elektronen durch eine durchtunnelbare Isolatorschicht und durch eine Elektronenemissionsschicht, und Beschleunigen der herausgelösten Elektronen mittels eines zweiten elektrischen Feldes, das durch Anlegen einer Spannung zwischen der Elektronenemissionsschicht und der wenigstens einen Elektrode erzeugt ist.

Vorzugsweise wird den herausgelösten Elektronen, beispielsweise durch ein inneres elektrisches Feld, eine Vorzugsrichtung der Emission aufgeprägt.

Die Erfindung betrifft also einen Gassensor nach dem Anspruch 1, wobei der Druckreduktionsbehälter gegenüber unkontrolliertem Gaseinlass abgedichtet ist, mindestens einen Gaseinlass aufweist, und der mindestens eine Gaseinlass eine gasdurchlässige Membran umfasst.

Vorzugsweise umfasst die mindestens eine gasdurchlässige Membran Polydimethylsiloxan (PDMS).

Günstigerweise weist die mindestens eine gasdurchlässige Membran eine Dicke von 10-50 µm auf.

Ferner kann vorgesehen sein, dass der Druckreduktionsbehälter eine Pumpeinrichtung umfasst.

Weiterhin kann vorgesehen sein, dass der Druck im Druckreduktionsbehälter im Betrieb des Gassensors 100 - 200 mbar beträgt.

In einer weiteren besonderen Ausführungsform der Erfindung weist die Ionendetektionseinrichtung einen oder mehrere Faraday-Becher auf.

Schließlich kann die Ionendetektionseinrichtung einen oder mehrere Sekundärelektronenvervielfacher umfassen.

Der Erfindung liegt die überraschende Erkenntnis zu Grunde, dass Materialien mit niedrigen Austrittsarbeiten durch geeignete Metalllegierungen erzeugt werden können und aus diesen herausgelöste Elektronen durch eine geeignete Elektronenbeschleunigungsstruktur auf geringe Energien mit geringer Energiebreite beschleunigt werden können. Als geringe Energien werden in diesem Zusammenhang vorzugsweise Energien im Bereich unter 25 eV, besonders bevorzugt im Bereich von unter 10 eV verstanden. Als geringe Energiebreite wird vorzugsweise eine Energiebreite von unter 1 eV, besonders bevorzugt von unter 0,5 eV und am bevorzugtesten von unter 0,1 eV verstanden.

Weitere Merkmale und Vorteile der Erfindung ergeben sich aus den beigefügten Ansprüchen und der nachfolgenden Beschreibung, in der anhand der schematischen Zeichnungen ein Ausführungsbeispiel der vorliegenden Erfindung beschrieben wird. Dabei zeigt:
- Figur 1: eine Schnittansicht von einem Äußerer-Photoeffekt-Emitter, der nicht Teil der vorliegenden Erfindung ist;
- Figur 2: eine Schnittansicht von einem Tunnel-Flächenemitter, der in den erfindungsgemäßen Gassensor integriert wird;
- Figur 3: eine Schnittansicht eines halbleiterbasierten Direktemitters, der nicht Teil der vorliegenden Erfindung ist;
- Figur 4: eine Schnittansicht eines weiteren halbleiterbasierten Direktemitters, der nicht Teil der vorliegenden Erfindung ist;
- Figur 5: eine Schnittansicht eines Flüssigkeitsionisators, der nicht Teil der vorliegenden Erfindung ist;
- Figur 6: eine Schnittansicht einer Partikelsammelvorrichtung, die nicht Teil der vorliegenden Erfindung ist,
- Figur 7: eine Schnittansicht eines Gassensors mit einem Tunnel-Flächenemitter gemäß einer besonderen Ausführungsform der Erfindung.

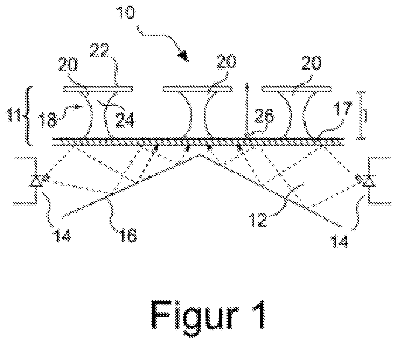

Figur 1 zeigt eine Schnittansicht von einem Äußerer-Photoeffekt-Emitter 10. Der Äußerer Photoeffekt-Emitter 10 umfasst eine Elektronenemitterstruktur 11, wobei die Elektronenemitterstruktur 11 wiederum eine atmosphärenstabile Elektronenemissionsschicht 17 aus einem Gemisch von Metallen mit einer ersten Seite und einer zweiten Seite, und eine Elektronenbeschleunigungsstruktur 18 auf der ersten Seite der Elektronenemissionsschicht 17 umfasst. Die Elektronenemissionsschicht 17 weist eine Dicke zwischen 1 nm und 10 nm auf.

Die Elektronenbeschleunigungsstruktur 18 umfasst mehrere Elektronenbeschleunigungselemente 20. Die Elektronenbeschleunigungsstruktur 18 ist dazu geeignet, geladene Teilchen, insbesondere Elektronen, zu beschleunigen. Die Elektronenbeschleunigungselemente 20 befinden sich oberhalb der Elektronenemissionsschicht 17 und sind mit dieser verbunden. Die Elektronenbeschleunigungselemente 20 weisen ihrerseits jeweils eine Elektrode 22 und ein elektrisch isolierendes Strukturelement 24 auf. Vorzugsweise befindet sich das Strukturelement 24 in direktem Kontakt oberhalb der Elektronenemissionsschicht 17. Im Betrieb werden die Elektroden 22 über eine Spannungsquelle (nicht gezeigt) positiv oder negativ gegenüber der Elektronenemissionsschicht 17 geladen. Optional kann die Elektronenbeschleunigungsstruktur 18 Deckelektroden (nicht gezeigt) oberhalb der Elektroden 22 aufweisen. Durch Anlegen von einem Nullpotential oder einem Abstoßungspotential lässt sich der Bereich oberhalb der Elektroden 22 feldfrei einstellen, was eine freie Bewegung der Elektronen 26 in einem an die Elektroden 22 angrenzenden Ionisationsraum (nicht explizit gezeigt) gewährleistet.

Ferner könnte die Elektronenbeschleunigungsstruktur eine zylinderförmige Steuerelektrode (nicht gezeigt) aufweisen. Eine solche zylinderförmige Steuerelektrode könnte in Form eines Wehneltzylinders aufgebaut sein. Mit der zylinderförmigen Steuerelektrode lassen sich die Elektronen auf Bereiche zwischen den Elektronenbeschleunigungselemente fokussieren. Hierdurch lässt sich unter anderem eine Aufladung der elektrisch isolierenden Strukturelemente 24 verhindern.

Der Äußerer-Photoeffekt-Emitter 10 umfasst ferner ein Substrat 12 aus einem UVtransparenten Material mit einer Vorderseite und eine Rückseite. Neben dem Substrat 12 sind UV-LEDs 14 derart angeordnet, dass sie Licht, dargestellt als gestrichelte Pfeile, in Richtung des transparenten Substrats 12 emittieren. Alternativ können die UV-LEDs 14 auch in dem Substrat 12 integriert sein. Die UV-LEDs 14 emittieren vorzugsweise Licht einer Wellenlänge zwischen 240 nm und 400 nm. Auf der Rückseite des Substrats 12 ist eine reflektierende Schicht 16 angeordnet, die geeignet ist, das von den UV-LEDs 14 emittierte Licht zu reflektieren. Die reflektierende Schicht 16 weist dazu vorzugsweise ein Material mit sehr hoher Austrittsarbeit auf. Eine sehr hohe Austrittsarbeit bedeutet in diesem Zusammenhang vorzugsweise eine Austrittsarbeit von mehr als 4,5 eV, besonders bevorzugt von mehr als 5 eV. Das Material kann hierzu beispielsweise Platin, Quecksilber, Nickel, Palladium und Iridium aufweisen. Vorzugsweise ist die von der Elektronenemissionsschicht 17 abgewandte Seite des Substrats 12 derart strukturiert, dass das von den UV-LEDs 14 emittierte Licht auch mehrfach in Richtung der Elektronenemissionsschicht 17 reflektiert werden kann. Hierzu eignen sich beispielsweise Flächen, die einen Winkel zur Horizontalen aufweisen. Durch die oben genannte Mehrfachreflexion treffen nicht absorbierte Photonen mehrfach auf Elektronenemissionsschicht 17, wodurch die Absorptionswahrscheinlichkeit erhöht und die Emissionseffizienz des Äußerer-Photoeffekt-Emitters verstärkt wird.

Im Betrieb des Äußerer-Photoeffekt-Emitters 10 emittieren die UV-LEDs 14 hochenergetische Photonen im UV-Bereich mit Wellenlängen zwischen etwa 240 nm bis 400 nm. Die Photonen treffen entweder direkt oder nach Reflexion von der reflektierenden Schicht 16 auf die Elektronenemissionsschicht 17. Die Photonen werden von der Elektronenemissionsschicht 17 absorbiert und lösen aus dieser durch den äußeren Photoeffekt Elektronen 26 aus. Da die Eindringtiefe der Photonen typischerweise größer ist als die mittlere freie Weglänge der Elektronen 26 ist, ist ein streifender Einfall des Lichts vorteilhaft, um eine ausreichende Anzahl an freien Elektronen 26 zu erzeugen. Der streifende Einfall wird durch die Anordnung der UV-LEDs 14 seitlich neben dem Substrat begünstigt. Die Elektronenbeschleunigungsstruktur 18 erzeugt oberhalb der Elektronenemissionsschicht 17 über eine zwischen den Elektroden 22 und der Elektronenemissionsschicht 17 angelegte Spannung ein elektrisches Feld, das im Wesentlichen senkrecht zur Oberfläche der Elektronenemissionsschicht 17 verläuft und die Elektronen 26 beschleunigt. In Zusammenhang mit den Elektronen 26 soll der Begriff der Beschleunigung ausdrücklich eine positive und negative Beschleunigung umfassen. Allgemein lässt sich mittels der Elektronenbeschleunigungsstruktur 18 die kinetische Energie der emittierten Elektronen 26 über eine an den Elektroden 22 angelegte Beschleunigungsspannung einstellen. Die Elektronenemissionsschicht 17 besteht aus einem Material, das dazu geeignet ist, Elektronen durch den äußeren Photoeffekt zu emittieren und weist vorzugsweise eine geringe Austrittsarbeit, beispielsweise im Bereich zwischen 2,5 bis 3,6 eV, auf. Die Länge l der Beschleunigungsstrecke liegt dabei in der gleichen Größenordnung oder ist kleiner als die mittlere freie Weglänge der Gasmoleküle. Sie beträgt vorzugsweise 10 nm bis 1 µm, besonders bevorzugt 30 nm bis 300 nm.

Das Gemisch von Metallen kann beispielsweise eine Legierung, umfassend zwei oder mehr Elemente, ausgewählt aus As, Ag, Zn, Au, Pt, Ru Rh, Pd, Os, Ir, Ce, Zn, Bi, Te, Sm, Eu, Gd, Yt, Yb, Nd, Pr und La umfassen. Insbesondere sind Legierungen umfassend InCe, AsCeSm, AgSm, AgCe, AgSmCe und AgAsCeSm geeignet, die Elektronenemissionsschicht zu bilden. Alternativ kann das Gemisch von Metallen ein Eutektikum aufweisen oder aus diesem bestehen. Geeignete Eutektika bestehen aus mindestens einem Edelmetall, mindestens einem Lanthanid und mindestens einem Element ausgewählt aus, As, Te, Bi und Sn.

Das Gemisch von Metallen weist vorzugsweise eine niedrige Austrittsarbeit von unter 3,5 eV, besonders bevorzugt von unter 3,0 eV auf und wird erfindungsgemäß durch geeignete Legierungen erzeugt. Die exemplarischen Verbindungen InCe, AsCeSm, AgSm, AgCe, AgSmeCe und AgAsCeSm weisen dauerhaft (14 Monate Lagerung unter Normalbedingungen) konstante Austrittsarbeiten zwischen 1,2 eV und 3,2 eV auf.

Die verallgemeinerte Lehre zu dieser Materialklasse ist, dass die Edelmetalle und die "gediegen" vorkommenden Metalle insofern zu den Legierungseigenschaften beitragen, dass sie die Atmosphärenstabilität der Legierung erhöhen, während die Lanthanide die Austrittsarbeit der Legierung verringern. Liegt das Material dazu in einheitlicher Stöchiometrie vor, lässt es sich mit größerer kristalliner Ordnung abscheiden, als wenn das Material mit uneinheitlicher Stöchiometrie vorliegt. Hierdurch können die Edelmetallatome naheliegende Lanthanidatome vor einer Oxidation an Luft oder einer Reaktion mit der Luftfeuchtigkeit besser schützen. Die erhöhte Stabilität lässt sich mit dem sogenannten Spillover-Effekt erklären. In den hier genannten Materialgruppen werden Elektronen aus den f-Orbitalen der Edelmetallatome in die d-Orbitale der Lanthanidatome übertragen. Insbesondere ist eine einheitliche Stöchiometrie des Materials AgCeSm vorteilhaft für die Optimierung der niedrigen Austrittsarbeit und der Stabilität gegenüber der Atmosphäre. Hierfür eignen sich insbesondere die Verbindungen Ag₂(CeₓSm₁₋ₓ) und Ag(CeₓSm₁₋ₓ). Diese sind aber nur exemplarisch für eine Vielzahl von auf diesen Elementen basierenden Verbindungen genannt.

Alternativ oder zusätzlich ist es auch möglich, die Elektronenemissionsschicht 17 von der Vorderseite zu beleuchten, um Elektronen aus dieser auszulösen. Hierzu ist eine Elektronenbeschleunigungsstruktur 18 aus optisch transparenten Materialien effizienzsteigernd. Die isolierenden Strukturelemente 24 können hierzu beispielsweise aus Quarz oder einem Fluoridmaterial bestehen. Die Elektrode kann hierzu beispielsweise aus transparentem Indiumzinnoxid (ITO) bestehen. Die UV-LEDs sind in diesem Fall oberhalb der Elektronenemissionsschicht 17 angeordnet.

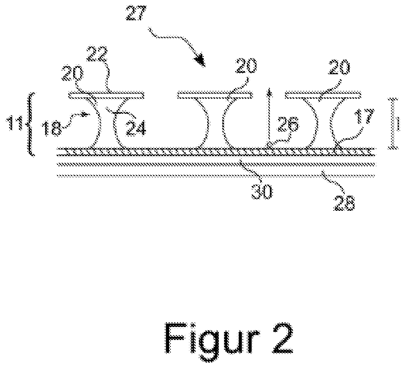

Figur 2 zeigt eine Schnittansicht von einem Tunnel-Flächenemitter 27, der in den erfindungsgemäßen Gassensor integriert wird. Der Tunnel-Flächenemitter 27 weist eine Elektronenemitterstruktur 11 beispielsweise gemäß der in Figur 1 gezeigten Ausführungsform der Erfindung, eine auf der zweiten Seite der Elektronenemissionsschicht 17 angeordnete Isolatorschicht 30 und eine auf einer der Elektronenemissionsschicht 17 abgewandten Seite auf der Isolatorschicht 30 angeordnete Metallschicht 28 auf. Die Schichtdicken der Metallschicht 28, der durchtunnelbaren Isolatorschicht 30 und der durchtunnelbaren Elektronenemissionsschicht 17 betragen vorzugsweise jeweils zwischen 0,5 nm und 6 nm, besonders bevorzugt zwischen 1 nm und 3 nm. Sowohl die Metallschicht 28 als auch die Elektronenemissionsschicht 17 sind leitfähig. Durch die dünne Isolatorschicht 30 zwischen den beiden leitfähigen Schichten lassen sich durch Anlegen einer Spannung starke elektrische Felder innerhalb dieser Schichten erzeugen. In den drei genannten Schichten befindliche Elektronen können Energie aus dem so erzeugten elektrischen Feld aufnehmen, sodass deren mittlere Energie über dem der Gittertemperatur entsprechenden Gleichgewichtswert liegt. Derart erzeugte sogenannten "heißen Elektronen" können aufgrund ihrer hohen Energie aus der durchtunnelbaren Anode 32 und der Elektronenemissionsschicht 17 emittieren. Der Tunnel-Flächenemitter 27 weist oberhalb der durchtunnelbaren Anode 32 eine Elektronenbeschleunigungsstruktur 18 entsprechend der in Zusammenhang mit Figur 1 beschriebenen besonderen Ausführungsform der Erfindung auf. Diese Elektronenbeschleunigungsstruktur 18 ist in gleicher Weise wie bei dem Äußerer-Photoeffekt-Emitter 10 aus Figur 1 dazu in der Lage, die emittierten Elektronen 26 zu beschleunigen bzw. abzubremsen.

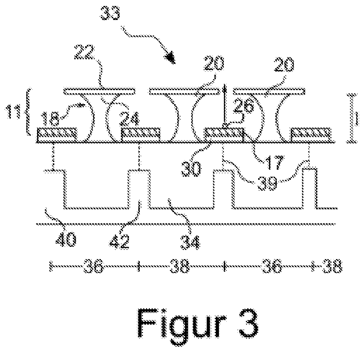

Figur 3 zeigt einen Querschnitt eines halbleiterbasierten Direktemitters 33. Der halbleiterbasierte Direktemitter 33 umfasst eine Elektronenemitterstruktur 11 beispielsweise gemäß dem in Figur 1 gezeigten Beispiel, eine Isolatorschicht 30, die auf der zweiten Seite der Elektronenemissionsschicht 17 angeordnet ist und eine durchtunnelbare Schichtdicke aufweist, und einen auf einer der Elektronenemissionsschicht 17 abgewandten Seite der Isolatorschicht 30 angeordneten Halbleiter 34, der horizontal nebeneinanderliegende n-dotierte Bereiche 36 und p-dotierte Bereiche 38 aufweist. Der Halbleiter 34 weist eine direkte, große Bandlücke von mindestens 3,0 eV auf. Der Halbleiter kann hierzu beispielsweise AlGaN oder InAlGaN umfassen. An den durch vertikale gestrichelte Linien gezeigten Grenzbereichen (39) zwischen den n- und p-dotierten Bereichen 36, 38 befinden sich jeweils p-n-Übergänge. Die p-n-Übergänge eignen sich zur Emission von Photonen, durch Rekombination von Elektronen und Löchern, beispielsweise in einem Quantenfilm (hier nicht explizit gezeigt), der eine niedrigere Bandlücke als das umgebende Halbleitermaterial 34 aufweist. Auf diese Weise bildet das Halbleitermaterial 34 eine Leuchtdiode an jedem der p-n-Übergänge. An einer Unterseite grenzt das Halbleitermaterial 34 an einen Isolator 40. Der Isolator 40 weist an den Stellen der p-n-Übergänge in das Halbleitermaterial 34 ragende Finger 42 auf. Die Finger 42 verringern die Größe der p-n-Übergänge in der Leuchtdiode bei annähernd gleichbleibender Größe der n- und p-dotierten Bereiche 36, 38. Durch die derartige Einschränkung der p-n-Übergänge lässt sich die Ladungsträgerdichte an den p-n-Übergängen erhöhen.

Auf einer ersten Seite des Halbleitermaterials 34 befinden sich Elemente bestehend aus einer durchtunnelbaren Isolatorschicht 30 und einer Elektronenemissionsschicht 17 in ähnlicher oder gleicher Weise wie in den Figuren 1 und 2. Ferner weist der halbleiterbasierte Direktemitter 33, ebenfalls wie in den Figuren 1 und 2 dargestellt, eine Elektronenbeschleunigungsstruktur 18 auf, mit der sich aus der Elektronenemissionsschicht 17 ausgelöste Elektronen beschleunigen lassen.

Sowohl die durchtunnelbare Isolatorschicht 30 und die Elektronenemissionsschicht 17 können jeweils Schichtdicken zwischen 0,5 nm und 5 nm aufweisen. Die hier gezeigte unterbrochene Elektronenemissionsschicht 17 zeigt lediglich eine besondere Ausführungsform der Erfindung. Eine ununterbrochene Isolatorschicht 30 und Elektronenemissionsschicht 17 kann ebenfalls genutzt werden.

Wird eine der Leuchtdioden durch Anlegen einer Spannung betrieben, werden freie Elektronen und Löcher im Leitungsband bzw. im Valenzband des Halbleitermaterials. Wird außerdem an der über dem Grenzbereich 39 der betriebenen Leuchtdiode angeordneten Elektronenemissionsschicht 17 eine positive Spannung angelegt, wird ein im Grenzbereich 39 der Leuchtdiode erzeugtes Elektron 26 in Richtung der Elektronenemissionsschicht 17 beschleunigt. Wird das Elektronen 26 im Grenzbereich 39 ausreichend nahe an der Isolatorschicht 17 erzeugt und in Richtung dieser beschleunigt, besteht die Möglichkeit, dass eine Rekombination verhindert wird. Das Elektron 26 kann stattdessen, aufgrund deren geringen Schichtdicken, sowohl die Isolatorschicht 17, als auch die Elektronenemissionsschicht 17 durchtunneln. Auf diese Weise kann ein Strom von freien Elektronen außerhalb der Leuchtdiode erzeugt werden.

Ferner kann der halbleiterbasierte Direktemitter 33, ebenfalls wie in den Figuren 1 und 2 dargestellt, eine Elektronenbeschleunigungsstruktur 18 aufweisen, mit der sich aus der Elektronenemissionsschicht 17 ausgelöste Elektronen 26 beschleunigen lassen. Diese ist für einen derartigen halbleiterbasierten Direktemitter 33 jedoch grundsätzlich nur optional. Die Elektronenbeschleunigungsstruktur 18 kann grundsätzlich, je nach Anwendungsbereich des halbleiterbasierten Direktemitters 33, auch entfallen.

Zur Strombegrenzung des Emitters, um einen Durchbruch zu vermeiden umfasst die Elektronenemissionsschicht 17 in dieser Ausführungsform vorzugsweise Halbmetalle, beispielsweise Arsen, Selen, Antimon, Tellur und Wismut. Für Verbindungen aus diesen Elementen reichen die zur Beschleunigung hilfreichen Raumladungszonen tiefer als bis zur ersten Atomlage. Alternativ kann für diesen Zweck auch ein perowskitisches Material, also ein Material mit einer Vorzugsachse des k-Vektors eingesetzt werden. In der emissiven Schicht ist die Impulsverteilung der angeregten Elektronen grundsätzlich unidirektional. Durch mechanischen Stress in der Ebene wird eine vertikale Vorzugsrichtung der Impulsverteilung erzeugt. Hierzu ist hier eine perowskitische Gitterstruktur besonders geeignet.

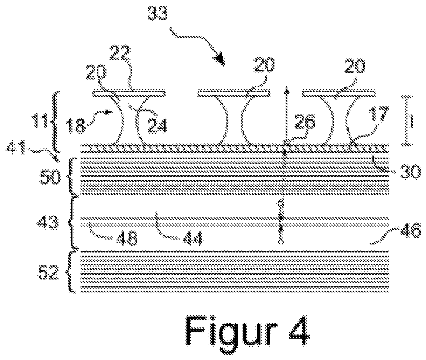

Figur 4 zeigt eine Schnittansicht eines weiteren halbleiterbasierten Direktemitters 33. Dieses Beispiel weist viele der in Zusammenhang mit Figur 2 beschriebenen Elemente auf. Die mit gleichen Bezugszeichen bezeichneten Elemente entsprechen den in Figur 2 gezeigten. Insbesondere weist der halbleiterbasierte Direktemitter 33 ebenfalls eine Elektronenemitterstruktur 11 beispielsweise gemäß dem in Figur 1 gezeigten Beispiel und eine Isolatorschicht 30 auf. Auf einer der Elektronenemissionsschicht 17 abgewandten Seite der Isolatorschicht ist ein oberflächenemittierender Laser mit vertikaler Kavität (vertical cavity surface emitting laser, VCSEL) 41 angeordnet. Der VCSEL 41 weist eine Kavität 43, einen oberen Bragg-Spiegel 50 und einen unteren Bragg-Spiegel 52 auf. Die Kavität 43 weist ihrerseits eine n-dotierte Schicht 44, eine p-dotierte Schicht 46 und einen Quantenfilm 48 auf. Der obere und untere Bragg-Spiegel 50, 52 befinden sich jeweils in direktem Kontakt zur Kavität 43. Die Bandlücke des Quantenfilms 48 ist kleiner als die der sie umgebenden n- und p-dotierten Schichten 44, 46.

Im Betrieb des halbleiterbasierten Direktemitters 33 rekombinieren Elektronen aus der n-dotierten Schicht 44 mit Löchern aus der p-dotierten Schicht 46 im Quantenfilm 48 und erzeugen so Photonen geeigneter Wellenlänge, die von den Bragg Spiegeln 50, 52 mehrfach reflektiert wird und durch stimulierte Emission weitere Photonen aus dem Quantenfilm 48 auslösen. Der obere Bragg-Spiegel 50 weist eine geringere Reflektivität als der untere Bragg-Spiegel 52 auf. Hierdurch emittiert der VCSEL 41 Photonen in Richtung der Elektronenemissionsschicht 17. Durch den Photoeffekt wird ein Elektron 26 in der Elektronenemissionsschicht 17 auslöst. Das Elektron kann anschließend durch die Elektronenbeschleunigungsstruktur 18 beschleunigt oder abgebremst werden. Die Prozesse sind schematisch durch die durchgezogenen und gestrichelten Pfeile dargestellt. Alternativ zum VCSEL 41 kann auch ein seitlich emittierender Halbleiterlaser oder auch eine Leuchtdiode verwendet werden.

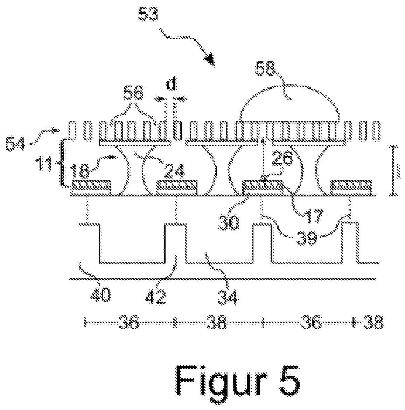

Figur 5 zeigt eine Schnittansicht eines Flüssigkeitsionisators 53. Der Flüssigkeitsionisators 53 dient zur Ionisation von Flüssigkeiten bzw. zur Lieferung von Elektronen 26 einer definierten Energie in Flüssigkeiten. Der Flüssigkeitsionisators 53 umfasst hierzu einen halbleiterbasierten Direktemitter 33, beispielsweise gemäß dem in Figur 3 gezeigten Beispiel auf. Alternativ kann aber beispielsweise auch jede der Beispiele gemäß den Figuren 1,2 oder 4 als Basis für den Flüssigkeitsionisator 53 dienen. Der Flüssigkeitsinitiator 53 weist eine auf einer der Elektronenemissionsschicht 17 abgewandten Seite der Elektronenbeschleunigungsstruktur 18 angeordnete Schutzschicht 54 auf. Die Schutzschicht 54 erlaubt die Emission von Elektronen 26 durch in der Schutzschicht 54 befindliche Löcher 56. Die Löcher 56 weisen einen Durchmesser d auf, der so gewählt ist, dass eine auf die Schutzschicht 54 aufgebrachte Flüssigkeit 58 aufgrund ihrer Oberflächenspannung nicht durch die Löcher durchdringt und die Elektronenemission möglichst wenig eingeschränkt ist. Daneben dient die Schutzschicht 54 dem Schutz der Elektroden 22 und der Elektronenemissionsschicht 17 vor Reaktionen mit der Flüssigkeit 58. Die Schutzschicht besteht vorzugsweise aus einem isolierenden Polymer, wie beispielsweise Fluorcarbon oder Fluorsilikonschichten. Die beiden letztgenannten Materialien verstärkten unter anderem den flüssigkeitsabweisenden Lotosblüteneffekt. Der Durchmesser d der Löcher 56 beträgt vorzugsweise zwischen 10 µm und 100 µm, besonders bevorzugt zwischen 20 µm und 50 µm. Die Elektronen emittieren, wie oben beschrieben, von der Elektronenemissionsschicht 17 weg, werden durch die Elektronenbeschleunigungsstruktur 18 beschleunigt oder abgebremst, fliegen durch die Löcher 56 der Schutzschicht 54 und treffen auf die Oberfläche der Flüssigkeit 58. Die Elektronenemission auf Flüssigkeitsoberflächen führt lokal zu starken Änderungen der Oberflächenenergien. Hierdurch werden Elektronen nicht mehr diffusiv mit einem breiten Energiespektrum, sondern mit Energien, wie sie exakt für einen bestimmten Reaktionsverlauf benötigt werden, bereitgestellt. Dies ist z.B. bei der Katalyse oder elektrochemischen Reaktionen hilfreich. Hierdurch werden Reaktionsverläufe gezielter steuerbar als es bisher mit Katalysatoren oder Morphologien reaktiver Oberflächen möglich ist.

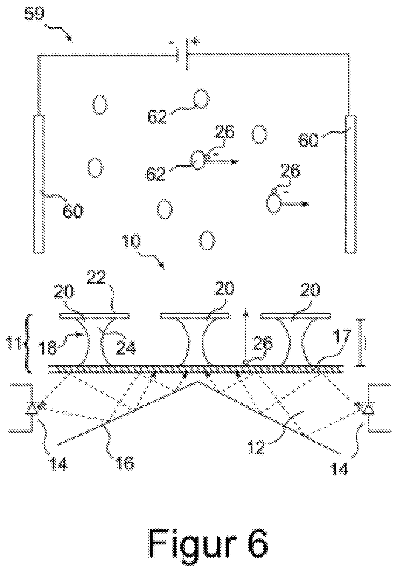

Figur 6 zeigt eine Partikelsammelvorrichtung 59 und kann beispielsweise, wie hier gezeigt in Partikel 62 enthaltender Luft eingesetzt werden. Die Partikelsammelvorrichtung 59 umfasst einen Äußerer-Photoeffekt-Emitter 10, beispielsweise entsprechend dem in Figur 1 gezeigten Beispiel. Alternativ kann aber beispielsweise auch jede der Beispiele gemäß den Figuren 2,3 oder 4 als Basis für den Flüssigkeitsionisator 53 dienen. Auf der ersten Seite der Elektronenemissionsschicht 17 in einem Abstand zueinander und jeweils senkrecht zur Elektronenemissionsschicht 17sind zwei mit unterschiedlicher Polarität geladene Platten 60 angeordnet. Die Platten 60sind dazu geeignet, ein elektrisches Feld senkrecht zur Emissionsrichtung der Elektronen 26 zu erzeugen. Von dem Äußerer-Photoeffekt-Emitter 10 emittierte Elektronen 26 können im Betrieb der Partikelsammelvorrichtung 59 von zwischen den Platten 60 befindlichen Partikeln 62 eingefangen werden. Auf diese Weise werden die Partikel 62 selbst negativ geladen. In dem von den Platten 60 erzeugten elektrischen Feld werden die negativ geladenen Partikel 62 in Richtung der positiv geladenen Platte 60 beschleunigt und können entweder von der Platte 60 selbst, oder von einem vor der Platte 60 befindlichen Filter (nicht gezeigt) eingefangen werden. Auf diese Weise werden sie der Luft entzogen.

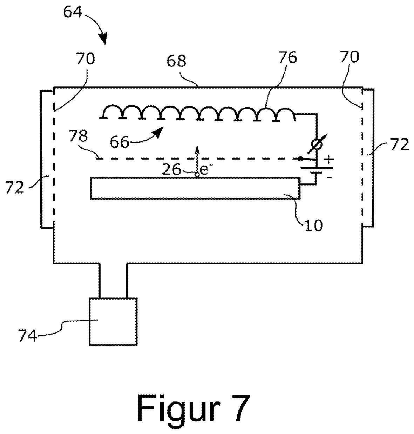

Figur 7 zeigt eine Schnittansicht eines Gassensors 64. Der Gassensor 64 ist dazu eingerichtet, in einer Gasatmosphäre betrieben zu werden, und weist einen Äußerer-Photoeffekt-Emitter 10, eine Ionendetektionseinrichtung 66 und einen Druckreduktionsbehälter 68 auf. Diese Variante mit dem Äußerer-Photoeffekt-Emitter 10 ist nicht Teil der vorliegenden Erfindung. Der Druckreduktionsbehälter 68 ist hierzu gegenüber unkontrolliertem Gaseinlass abgedichtet. Der Äußerer-Photoeffekt-Emitter 10 und die Ionendetektionseinrichtung 66 sind innerhalb des Druckreduktionsbehälters 68 angeordnet. Der Gassensor 64 ist dazu eingerichtet, den Druck innerhalb des Druckreduktionsbehälters 68 gegenüber einem Druck außerhalb des Druckreduktionsbehälter 68 zu reduzieren. Hierzu ist am Druckreduktionsbehälter 68 eine Pumpeinrichtung 74 angeordnet, die im Betrieb des Gassensors Gas aus dem Druckreduktionsbehälter 68 abpumpt. Die Pumpeinrichtung 74 kann beispielsweise eine Membranpumpe oder eine andere geeignete Pumpeinrichtung 74 sein, die geeignet ist, den Druck im Inneren des Druckreduktionsbehälters 68 zu reduzieren.

Der Druckreduktionsbehälter 68 weist vorzugweise mindestens einen Gaseinlass 70 zum kontrollierten Einlass von Gas in den Druckreduktionsbehälter 68 auf. Der Druck im Innern des Druckreduktionsbehälters 68 kann durch die Pumpleistung der Pumpeinrichtung 74 und die Größe des Gaseinlasses 70 kontrolliert werden. Im Betrieb des Gassensors 64 ist der Druck im Druckreduktionsbehälter 68 relativ zum Umgebungsdruck grundsätzlich reduziert.

Alternativ oder zusätzlich können ein oder mehrere Ventile (hier nicht dargestellt) zur Kontrolle des Drucks beispielsweise am Gaseinlass 70 und/oder an einem Eingang der Pumpeinrichtung 74 angebracht sein. In der in Figur 7 gezeigten besonderen Ausführungsform der Erfindung sind zwei Gaseinlasse 70 jeweils mit einer gasdurchlässigen Membran 72 abgedeckt. Vorzugsweise umfasst die gasdurchlässige Membran 72 ein Material, das für Spurengase zumindest teilweise durchlässig ist, für Luftfeuchtigkeit aber nicht durchlässt und/oder absorbiert. Hierdurch kann unter anderem die Stabilität der Elektronenemissionsschicht 17 des Äußerer-Photoeffekt-Emitters 10 verbessert werden.

Ein für diesen Zweck geeignetes Material ist beispielsweise Polydimethylsiloxan (PDMS). Die gasdurchlässige Membran 74 kann PDMS umfassen oder aus PDMS bestehen. Durch die Anordnung der ein oder mehreren Gaseinlasse 70 und der Pumpeinrichtung 74 am Druckreduktionsbehälter 68 lässt sich die Strömungsrichtung des Gases innerhalb des Druckreduktionsbehälters 68 regeln.

Vorzugsweise beträgt der Druck im Druckreduktionsbehälter 68 im Betrieb des Gassensors zwischen 50-500 mbar, besonders bevorzugt zwischen 100-200 mbar. Durch die Druckreduktion wird der Gassensor 64 aktiv durchströmt. Eine Änderung der Gaszusammensetzung außerhalb der Gassensor 64 wirkt sich damit auch innerhalb kurzer Zeit auf die Gaszusammensetzung innerhalb des Gassensors 64 aus.

Im Betrieb des Gassensors 64 werden aus dem Äußerer-Photoeffekt-Emitter 10 Elektronen 26 emittiert. Die emittierten Elektronen 26 können über eine optionale Beschleunigungsstruktur 78 auf eine bestimmte Energie beschleunigt werden. Anschließend treffen die Elektronen 26 auf Gasmoleküle, die ionisiert werden. Die ionisierten Gasmoleküle werden von der Ionendetektionseinrichtung 66 detektiert. Hierzu kann die Ionendetektionseinrichtung 66 einen oder mehrere Faraday-Becher 76 umfassen. Alternativ oder zusätzlich zu den Faraday-Bechern kann vorgesehen sein, dass die Ionendetektionseinrichtung 66 einen oder mehrere Sekundärelektronenvervielfacher (hier nicht dargestellt) umfasst.

Alternativ zum Äußerer-Photoeffekt-Emitter 10 wird erfindungsgemäß vorgesehen, dass der Gassensor 64 zur Emission von Elektronen einen Tunnel-Flächenemitter 27 aufweist. Die Funktionsweise des Gassensors 64 ist bei dieser Ausführungsform der Erfindung analog zu der in Zusammenhang mit Figur 7 beschriebenen Funktionsweise.

### Bezugszeichenliste

- 10: Äußerer-Photoeffekt-Emitter
- 11: Elektronenemitterstruktur
- 12: Substrat
- 14: UV-LED
- 16: reflektierende Schicht
- 17: Elektronenemissionsschicht
- 18: Elektronenbeschleunigungsstruktur
- 20: Elektronenbeschleunigungselement
- 22: Elektrode
- 24: Strukturelement
- 26: Elektron
- 27: Tunnel-Flächenemitter
- 28: Metallschicht
- 30: Isolatorschicht
- 32: durchtunnelbare Anode
- 33: halbleiterbasierter Direktemitter
- 34: Halbleiter
- 36: n-dotierter Bereich
- 38: p-dotierter Bereich
- 39: Grenzbereich
- 40: Isolator
- 41: VCSEL
- 42: Finger
- 43: Kavität
- 44: n-dotierte Schicht
- 46: p-dotierte Schicht
- 48: Quantenfilm
- 50: oberer Bragg-Spiegel
- 52: unterer Bragg-Spiegel
- 53: Flüssigkeitsionisator
- 54: Schutzschicht
- 56: Löcher
- 58: Flüssigkeit
- 59: Partikelsammelvorrichtung
- 60: Platte
- 62: Partikel
- 64: Gassensor
- 66: Ionendetektionseinrichtung
- 68: Druckreduktionsbehälter
- 70: Gaseinlass72 gasdurchlässige Membran
- 74: Pumpeinrichtung
- 76: Faraday-Becher
- 78: Beschleunigungsstruktur

- l: Länge der Beschleunigungsstrecke
- d: Durchmesser der Löcher

## Patentansprüche

1. Gassensor (64), umfassend
• einen Tunnel-Flächenemitter (27), mit einer Elektronenemitterstruktur,
wobei die Elektronenemitterstruktur (11)
- eine atmosphärenstabile Elektronenemissionsschicht (17) aus einem Gemisch von Metallen, wobei die atmosphärenstabile Elektronenemissionsschicht eine erste Seite und eine zweite Seite aufweist; und
- eine Elektronenbeschleunigungsstruktur (18) auf der ersten Seite der Elektronenemissionsschicht (17) mit wenigstens einer gegenüber der Elektronenbeschleunigungsstruktur (18) elektrisch isolierten Elektrode (22) zur Bildung einer Beschleunigungsstrecke, die konfiguriert ist, um zu ermöglichen, dass aus der Elektronenemissionsschicht (17) herausgelöste Elektronen (26) bei Erzeugen eines einstellbaren elektrischen Feldes gezielt beschleunigt werden, wobei die Beschleunigungsstrecke eine Länge I in einem Bereich von 10 nm bis 1 µm aufweist,
umfasst, und
wobei der Tunnel-Flächenemitter eine auf der zweiten Seite der Elektronenemissionsschicht (17) angeordnete Isolatorschicht (30), die eine Schichtdicke zwischen 0,5 nm und 6 nm aufweist, und eine auf einer der Elektronenemissionsschicht (17) abgewandten Seite auf der Isolatorschicht (30) angeordnete Metallschicht (28), die eine Schichtdicke zwischen 0,5 nm und 6 nm aufweist, wobei sowohl die Elektronenemissionsschicht (17) als auch die Isolatorschicht (30) durchtunnelbare Schichtdicken aufweisen, umfasst,
• eine lonendetektionseinrichtung (66); und
• einen Druckreduktionsbehälter (68), in dem der Tunnel-Flächenemitter (27) angeordnet ist, wobei
im Betrieb des Gassensors (64) der Druck im Druckreduktionsbehälter (68) relativ zum Umgebungsdruck reduziert ist.

2. Gassensor (64) nach Anspruch 1, wobei das Gemisch von Metallen eine Legierung ist, vorzugsweise umfassend eine Verbindung ausgewählt aus InCe, AsCeSm, AgSm, AgCe, AgSmCe und AgAsCeSm und/oder zwei oder mehr Elemente aus As, Ag, Zn, Au, Pt, Ru Rh, Pd, Os, Ir, Ce, Zn, Bi, Te, Sm, Eu, Gd, Yt, Yb, Nd, Pr und La.

3. Gassensor (64) nach Anspruch 1, wobei das Gemisch von Metallen ein Eutektikum, umfassend mindestens ein Edelmetall, mindestens ein Lanthanid und mindestens ein Element ausgewählt aus As, Te, Bi und Sn, umfasst.

4. Gassensor (64) nach einem der vorhergehenden Ansprüche, wobei die Elektronenbeschleunigungsstruktur (18) ferner wenigstens ein isolierendes Strukturelement (24) umfasst, das zusammen mit der wenigstens eine Elektrode (22) wenigstens ein Elektronenbeschleunigungselement (20) bildet.

5. Gassensor (64) Anspruch 4, wobei das wenigstens eine isolierende Strukturelement (24) zwischen der Elektronenemissionsschicht (17) und der wenigstens einen Elektrode (22) angeordnet und damit verbunden ist.

6. Gassensor (64) nach einem der vorstehenden Ansprüche, wobei die Elektronenbeschleunigungsstruktur (18) mehrere Elektronenbeschleunigungselemente (20) umfasst, die in einem Array, vorzugsweise lateral, angeordnet sind.

7. Gassensor (64) nach einem der vorstehenden Ansprüche, wobei der Druckreduktionsbehälter (68)
- gegenüber unkontrolliertem Gaseinlass abgedichtet ist,
- mindestens einen Gaseinlass (70) aufweist, und
- der mindestens eine Gaseinlass (70) eine gasdurchlässige Membran (72) umfasst.

8. Gassensor (64) nach Anspruch 7, wobei die mindestens eine gasdurchlässige Membran (72) Polydimethylsiloxan (PDMS) umfasst.

9. Gassensor (64) nach einem der Ansprüche 7 oder 8, wobei die mindestens eine gasdurchlässige Membran (72) eine Dicke von 10-50 µm aufweist.

10. Gassensor (64) nach einem der vorstehenden Ansprüche, wobei der Druckreduktionsbehälter (68) eine Pumpeinrichtung (74) umfasst.

11. Gassensor (64) nach einem der vorstehenden Ansprüche, wobei der Druck im Druckreduktionsbehälter (68) im Betrieb des Gassensors (64) 100 - 200 mbar beträgt.

12. Gassensor (64) nach einem der vorstehenden Ansprüche, wobei die lonendetektionseinrichtung (66) einen oder mehrere Faraday-Becher (76) aufweist.

13. Gassensor (64) nach einem der vorstehenden Ansprüche, wobei die lonendetektionseinrichtung (66) einen oder mehrere Sekundärelektronenvervielfacher umfasst.

## Claims

1. Gas sensor (64), comprising
- a surface tunnel emitter (27) with an electron emitter structure, wherein the electron emitter structure (11) comprises
- an atmosphere-stable electron emission layer (17) composed of a mixture of metals, wherein the atmosphere-stable electron emission layer comprises a first side and a second side; and
- an electron acceleration structure (18) on the first side of the electron emission layer (17) with at least one electrode (22) electrically insulated from the electron acceleration structure (18) to form an acceleration path which is configured to allow electrons (26) released from the electron emission layer (17) to be accelerated in a targeted manner upon generation of a controllable electric field, wherein the acceleration path has a length l in the range from 10 nm to 1 µm,
and
wherein the surface tunnel emitter comprises an insulating layer (30) arranged on the second side of the electron emission layer (17), which has a layer thickness of between 0.5 nm and 6 nm, and a metal layer (28) arranged on a side facing away from the electron emission layer (17) on the insulating layer (30), which has a layer thickness of between 0.5 nm and 6 nm, wherein both the electron emission layer (17) and the insulating layer (30) have layer thicknesses that can be tunnelled through,
- an ion detection device (66); and
- a pressure reduction container (68), in which the surface tunnel emitter (27) is arranged,
during operation of the gas sensor (64), the pressure in the pressure reduction container (68) is reduced relative to the ambient pressure.

2. Gas sensor (64) according to claim 1, wherein the metal mixture is an alloy, preferably comprising a compound selected from InCe, AsCeSm, AgSm, AgCe, AgSmCe and AgAsCeSm and/or two or more of As, Ag, Zn, Au, Pt, Ru Rh, Pd, Os, Ir, Ce, Zn, Bi, Te, Sm, Eu, Gd, Yt, Yb, Nd, Pr and La.

3. Gas sensor (64) of claim 1, wherein the metal mixture comprises a eutectic comprising at least one noble metal, at least one lanthanide and at least one element selected from As, Te, Bi and Sn.

4. Gas sensor (64) according to any one of the preceding claims, wherein the electron acceleration structure (18) further comprises at least one insulating structural element (24) which, together with the at least one electrode (22), forms at least one electron-accelerating element (20).

5. Gas sensor (64) according to claim 4, wherein the at least one insulating structural element (24) is arranged between and connected to the electron emission layer (17) and the at least one electrode (22).

6. Gas sensor (64) according to any one of the preceding claims, wherein the electron acceleration structure (18) comprises a plurality of electron-accelerating elements (20) arranged in an array, preferably laterally.

7. Gas sensor (64) according to any one of the preceding claims, wherein the pressure reduction container (68)
- is sealed off from an uncontrolled gas inlet,
- comprises at least one gas inlet (70), and
- the at least one gas inlet (70) comprises a gas-permeable membrane (72).

8. Gas sensor (64) according to claim 7, wherein the at least one gas-permeable membrane (72) comprises polydimethylsiloxane (PDMS).

9. Gas sensor (64) according to any one of claims 7 or 8, wherein the at least one gas-permeable membrane (72) has a thickness of 10 to 50 µm.

10. Gas sensor (64) according to any one of the preceding claims, wherein the pressure reduction container (68) comprises a pumping device (74).

11. Gas sensor (64) according to any one of the preceding claims, wherein the pressure in the pressure reduction container (68) is 100 to 200 mbar during operation of the gas sensor (64).

12. Gas sensor (64) according to any one of the preceding claims, wherein the ion detection device (66) comprises one or more Faraday cavities (76).

13. Gas sensor (64) according to any one of the preceding claims, wherein the ion detection device (66) comprises one or more secondary electron multipliers.

## Revendications

1. Capteur de gaz (64), comprenant
- un émetteur de surface à effet tunnel (27), avec une structure d'émetteur d'électrons, la structure d'émetteur d'électrons (11) comprenant
- une couche d'émission d'électrons stable à l'atmosphère (17) composée d'un mélange de métaux, la couche d'émission d'électrons stable à l'atmosphère présentant un premier côté et un deuxième côté ; et
- une structure d'accélération d'électrons (18) sur le premier côté de la couche d'émission d'électrons (17) avec au moins une électrode (22) isolée électriquement par rapport à la structure d'accélération d'électrons (18) pour former un trajet d'accélération qui est configuré pour permettre à des électrons (26) libérés de la couche d'émission d'électrons (17) d'être accélérés de manière ciblée lors de la génération d'un champ électrique réglable, le trajet d'accélération présentant une longueur l dans une plage de 10 nm à 1 µm,
et
l'émetteur de surface à effet tunnel comprenant une couche isolante (30) agencée sur le deuxième côté de la couche d'émission d'électrons (17), qui présente une épaisseur de couche comprise entre 0,5 nm et 6 nm, et une couche métallique (28) agencée sur un côté détourné de la couche d'émission d'électrons (17) sur la couche isolante (30), qui présente une épaisseur de couche comprise entre 0,5 nm et 6 nm, la couche d'émission d'électrons (17) et la couche isolante (30) présentant toutes deux des épaisseurs de couche pouvant être traversées par un effet tunnel,
- un dispositif de détection d'ions (66) ; et
- un réservoir de réduction de pression (68), dans lequel l'émetteur de surface à effet tunnel (27) est agencé,
lors du fonctionnement du capteur de gaz (64), la pression dans le réservoir de réduction de pression (68) étant réduite par rapport à la pression ambiante.

2. Capteur de gaz (64) selon la revendication 1, dans lequel le mélange de métaux est un alliage, de préférence comprenant un composé choisi parmi InCe, AsCeSm, AgSm, AgCe, AgSmCe et AgAsCeSm et/ou deux ou plus de deux éléments parmi As, Ag, Zn, Au, Pt, Ru Rh, Pd, Os, Ir, Ce, Zn, Bi, Te, Sm, Eu, Gd, Yt, Yb, Nd, Pr et La.

3. Capteur de gaz (64) selon la revendication 1, dans lequel le mélange de métaux comprend un eutectique comprenant au moins un métal noble, au moins un lanthanide et au moins un élément choisi parmi As, Te, Bi et Sn.

4. Capteur de gaz (64) selon l'une quelconque des revendications précédentes, dans lequel la structure d'accélération d'électrons (18) comprend en outre au moins un élément structurel isolant (24) qui, avec l'au moins une électrode (22), forme au moins un élément d'accélération d'électrons (20).

5. Capteur de gaz (64) selon la revendication 4, dans lequel l'au moins un élément structurel isolant (24) est agencé entre et relié à la couche d'émission d'électrons (17) et l'au moins une électrode (22).

6. Capteur de gaz (64) selon l'une quelconque des revendications précédentes, dans lequel la structure d'accélération d'électrons (18) comprend plusieurs éléments d'accélération d'électrons (20) agencés dans un réseau, de préférence latéralement.

7. Capteur de gaz (64) selon l'une quelconque des revendications précédentes, dans lequel le réservoir de réduction de pression (68)
- est scellé par rapport à une entrée de gaz non contrôlée,
- présente au moins une entrée de gaz (70), et
- l'au moins une entrée de gaz (70) comprend une membrane perméable aux gaz (72).

8. Capteur de gaz (64) selon la revendication 7, dans lequel l'au moins une membrane perméable aux gaz (72) comprend du polydiméthylsiloxane (PDMS).

9. Capteur de gaz (64) selon l'une quelconque des revendications 7 ou 8, dans lequel l'au moins une membrane perméable aux gaz (72) présente une épaisseur de 10 à 50 µm.

10. Capteur de gaz (64) selon l'une quelconque des revendications précédentes, dans lequel le réservoir de réduction de pression (68) comprend un dispositif de pompage (74).

11. Capteur de gaz (64) selon l'une quelconque des revendications précédentes, dans lequel la pression dans le réservoir de réduction de pression (68) est de 100 à 200 mbars pendant le fonctionnement du capteur de gaz (64).

12. Capteur de gaz (64) selon l'une quelconque des revendications précédentes, dans lequel le dispositif de détection d'ions (66) présente une ou plusieurs cavités de Faraday (76).

13. Capteur de gaz (64) selon l'une quelconque des revendications précédentes, dans lequel le dispositif de détection d'ions (66) comprend un ou plusieurs multiplicateurs d'électrons secondaires.
